(19) Europäisches Patentamt
European Patent Office
Office européen des brevets

(11) Numéro de publication: **0 189 448**
**B1**

# (12) FASCICULE DE BREVET EUROPÉEN

(45) Date de publication du fascicule du brevet: **25.01.89**

(51) Int. Cl.⁴: **C 07 B 39/00,** C 07 C 87/30

(21) Numéro de dépôt: **85903294.8**

(22) Date de dépôt: **27.06.85**

(86) Numéro de dépôt international: **PCT/FR 85/00175**

(87) Numéro de publication internationale: **WO 86/00294 (16.01.86 Gazette 86/02)**

## (54) AGENT DE FLUORATION DE COMPOSES ORGANIQUES NOTAMMENT DE COMPOSES ACETYLENIQUES, LEUR PROCEDE DE PREPARATION ET LEUR UTILISATION DANS LES REACTIONS D'ADDITION OU DE SUBSTITUTION NUCLEOPHILE.

(30) Priorité: **27.06.84 FR 8410137**

(43) Date de publication de la demande: **06.08.86 Bulletin 86/32**

(45) Mention de la délivrance du brevet: **25.01.89 Bulletin 89/4**

(84) Etats contractants désignés: **BE CH DE GB IT LI LU NL SE**

(56) Documents cité:
**FR-A-1 435 770**

**Chemical Abstracts, volume 93, no. 25, 22 décembre1980, Columbus, Ohio (US) A. Bensadat et al.:"Electrochemical preparation of benzylfluorides", voir page 808, abrégé 238946h, Nouv. J.Chim.1980 4(7)453-60**

(73) Titulaire: **UNIVERSITE D'ANGERS, 30, rue des Arènes, F-49035 Angers (FR)**

(72) Inventeur: **ALBERT, Patrice, Jean- Pierre, 45, rue de la Tour- Landry, F-49000 Angers (FR)**
Inventeur: **COUSSEAU, Jack, 12, allée des Noues Beaucouzé, F-49000 Angers (FR)**

(74) Mandataire: **Nony, Michel, Cabinet Nony 29, rue Cambacérès, F-75008 Paris (FR)**

**EP 0 189 448 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention a pour objet une nouvelle classe d'agents de fluoration de composés organiques, notamment de composés acétyléniques, leur procédé de préparation et leur utilisation dans les réactions d'addition ou de substitution nucléophile.

Les composés organo-fluorés présentent un grand intérêt notamment les composés fluorovinyliques dont certains tels que le fluorofumarate et ses dérivés qui manifestent d'intéressantes propriétés fongicides.

Par ailleurs, les composés fluorovinyliques constituent des intermédiaires précieux susceptibles de constituer des produits de départ pour diverses synthèses notamment pour la synthèse de polymères fluorés.

Les préparations jusqu'ici proposées de composés fluorovinyliques sont particulièrement longues et de ce fait peu économiques.

A ce jour, aucun agent de fluoration n'a été décrit comme susceptible d'additionner HF à des composés acétyléniques et ainsi conduire uniquement à des fluoro-oléfines.

En effet, les agents de fluoration connus tels que le fluorure d'hydrogène (HF) liquide dont la mise en oeuvre est d'ailleurs particulièrement délicate ainsi que d'autre réactifs tels que le réactif de G.A. OLAH (pyridine, 10 HF) J. Org. Chem. (1979), 44 (22) 3872 dont la réactivité est très similaire à celle de HF liquide, conduit à des dérivés fluorés saturés qui proviennent de la bis-addition de HF.

Il a également été proposé par ZUPAN et al Journal of Fluorine Chemistry, 24 (1984) 291 - 302, l'utilisation de résines porteuses de HF comme agents de fluoration. De telles résines sont particulièrement difficiles à mettre en oeuvre car leur préparation nécessite l'emploi de HF liquide et ces résines lorsqu'elles sont appliquées à des composés acétyléniques ne conduisent pas aux composés fluorovinyliques.

A la suite de travaux sur les ions hydrogènofluorures on a constaté que les ions du type [$F^-$, nHF] avec $n \simeq 2$ que l'on désignera ci-après par l'expression "anion dihydrogènotrifluorure" ou "$H_2F_3^-$" apparaissaient comme une bonne source de HF.

Les études réalisées ont en effet permis de montrer que les anions dihydrogènotrifluorures permettaient l'addition de HF à des triples liaisons bi-activées ou mono-activées conduisant ainsi à des composés mono-fluorés éthyléniques avec de bons rendements et qu'ils pouvaient également conduire à des réactions de substitution nucléophile.

Par ailleurs de façon assez surprenante et inattendue on a constaté que par rapport à certains agents de fluoration connus, les anions dihydrogènotrifluorures ne conduisaient pas à l'addition de HF sur les composés éthyléniques activés ou non activés.

L'état de la technique concernant les anions hydrogènofluorures comme agents de fluoration est essentiellement matérialisé d'une part par le brevet français n° 1 435 770 et d'autre part par l'article de BENSADAT et al, Nouv. J. Chim., 1980, 4(7), 753 - 60 qui décrivent l'utilisation de trihydrogénotétrafluorures dans la préparation de fluoralkyles et de fluoroaryles notamment de fluorures de benzyle.

La présente invention a pour objet à titre de produit industriel nouveau un agent de fluoration, notamment de composés acétyléniques, ledit agent de fluoration correspondant à la formule générale suivante:

$$Q^+ [F^-, n\,HF] \qquad (I)$$

dans laquelle:

$Q^+$ représente soit: (i) un cation de formule:

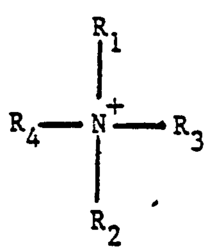

dans laquelle:

$R_1$, $R_2$, $R_3$ et $R_4$, identiques ou différents, représentent un radical alkyle ayant de 1 à 20 atomes de carbone, un radical aryle ou un radical aralkyle, le nombre d'atomes de carbone dans $R_1+R_2+R_3+R_4$ étant au moins égal à 13 et de préférence compris entre 15 et 22,

soit: (ii) une matrice polymérique porteuse de fonctions de formule:

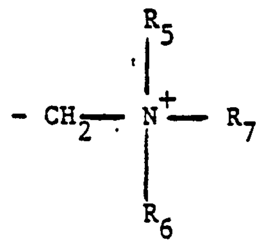

dans laquelle:

$R_5$, $R_6$ et $R_7$, identiques ou différents, représentent un radical alkyle inférieur ayant de 1 à 6 atomes de carbone,

et n est une valeur moyenne comprise entre 1,5 et 2,5 de préférence entre 1,8 et 2,1.

Comme on peut le constater d'après la formule ci-dessus le nombre "n" des composés de formule (I) est voisin de 2 de telle sorte que par mesure de simplification ces composés seront désignés ci-après par l'expression "dihydrogénotrifluorures".

Les "dihydrogénotrifluorures" tels que définis ci-dessus présentent l'avantage par rapport aux agents connus de permettre l'addition de HF à des triples liaisons activées et conduire ainsi de façon simple et économique à des composés fluorovinyliques.

Cette sélectivité vis à vis des liaisons acétyléniqucs est tout à fait remarquable et permet ainsi d'accéder à différents composés fluorovinyliques qui jusqu'à présent ne pouvaient être obtenus qu'au prix de synthéses particulièrement longues et avec un rendement peu élevé.

Par ailleurs, par rapport aux agents de fluoration connus, les agents selon l'invention sont facilement obtenus sans avoir recours à l'utilisation de fluorure d'hydrogène (HF) liquide, ce qui est particulièrement appréciable compte tenu du danger et des conditions particulières de manipulation de ce composé.

Selon une première forme de réalisation de l'invention, l'agent de fluoration peut être représenté par la formule suivante:

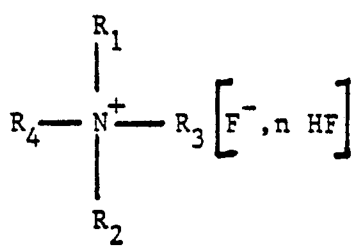

(II)

dans laquelle:

$R_1$, $R_2$, $R_3$ et $R_4$ identiques ou différents, représentent un radical alkyle ayant de 1 à 20 atomes de carbone, un radical phényle ou un radical benzyle, le nombre total d'atomes de carbone des radicaux $R_1+R_2+R_3+R_4$ étant au moins égal à 13 et de préférence compris entre 15 et 22,

et n est compris entre 1,5 et 2,5 et de préférence entre 1,8 et 2,1.

Parmi les dihydrogènotrifluorures correspondant à la formule (II) ci-dessus susceptibles d'être utilisés comme agents de fluoration, notamment de composés acétyléniques, on peut en particulier citer sans que cette énumération soit limitative:

le dihydrogènotrifluorure de tétrabutylammonium,
le dihydrogènotrifluorure de tétrapentylammonium,
le dihydrogènotrifluorure de tétrahéxylammonium,
le dihydrogènotrifluorure d'hexadécyltriméthylammonium, et
le dihydrogènotrifluorure de benzyltriéthylatmmonium.

Des résultats particulièrement intéressants ont été observés lorsque l'agent de fluoration est le dihydrogènotrifluorure de tétrabutylammonium.

Les différents dihydrogénotrifluorures d'ammonium de formule (II) présentent une bonne solubilité en milieu organique peu polaire notamment dans les solvants chlorés tels que le chloroforme, le tétrachlorure de

carbone, le dichlorotéthane ou le dichloro-1,2 éthane, ce qui facilite leur emploi non seulement dans les réactions d'addition mais également de substitution nucléophile.

Comme ceci ressort de la formule (II) donnée ci-dessus, le nombre d'atomes de carbone du cation doit être suffisamment élevé de façon à obtenir une bonne solubilité dans les solvants organiques.

En effet, on a observé que l'orsque le nombre d'atomes de carbone était inférieur à environ 13, les composés présentaient une certaine solubilité dans l'eau et une mauvaise solubilité dans les solvants peu polaires ou moyennement polaires.

Selon une deuxième forme de réalisation de l'invention, l'agent de fluoration selon l'invention peut être représenté par la formule suivante:

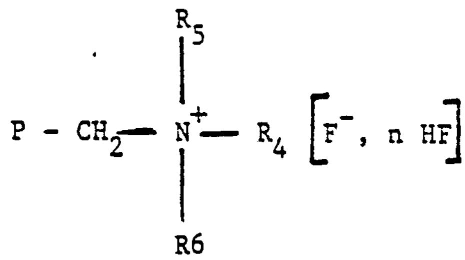

(III)

dans laquelle:

$R_4$, $R_5$ et $R_6$, identiques ou différents, réprésentent un radical alkyle inférieur ayant de 1 à 6 atomes de carbone et P représente une matrice polymérique notamment du type polystyrène-divinylbenzène,

et n est compris entre 1,5 et 2,5, de préférence entre 1,8 et 2,1.

Les matrices polymériques du type polystyrène divinylbenzène se rencontrent notamment dans des résines échangeuses d'anions. On peut à cet égard citer comme résines de ce type, les résines Amberlyst A 26 et Amberlite IRA 900.

Les différentes études réalisées à l'aide de ces résines porteuses d'anions dihydrogénotrifluorures ont permis de montrer qu'elles étaient susceptibles de conduire à des résultats tout à fait comparables aux composés de formule (II) et qu'elles présentaient par ailleurs le grand intérêt de pouvoir être facilement éliminées par filtration après réaction et être recyclées.

La présente invention a également pour objet le procédé de préparation des dihydrogènotrifluorures d'ammonium de formule (II).

Ce procédé qui est réalisé selon la technique de transfert de phase consiste à faire réagir une phase organique, constituée d'un solvant moyennement polaire tel que le dichlorométhane ou le dichloro-1,2 éthane contenant un fluorure d'ammonium de formule:

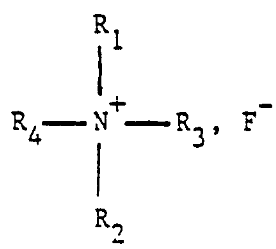

en présence d'une phase aqueuse saturée d'un mélange de fluorure d'hydrogène aqueux et d'un fluorure alcalin ou d'un hydrogénodifluorure alcalin, le rapport molaire des constituants de la phase aqueuse par rapport au fluorure d'ammonium étant compris entre environ 4 et 6.

La contentration du fluorure d'ammonium dans la phase organique revêt une importance particulière ceci de façon à ce que le nombre n reste compris dans les valeurs mentionnées ci-dessus c'est-à-dire entre 1,5 et 2,5 et de préférence entre 1,8 et 2,1.

De préférence la concentration du fluorure d'ammonium doit être comprise entre 0,08 et 0,12 mole par litre de solvant.

Les sels alcalins de la phase aqueuse sont de préférence ceux de potassium ou de sodium. On peut également remplacer l'hydrogénodifluoture alcalin par l'hydrogénodifluorure d'ammonium.

Comme ceci a été mentionné precédemment, ce procédé par rapport aux procédés connus présente le grand avantage de ne pas nécessiter l'utilisation de HP liquide-particulièrement délicat et dangereux à manipuler mais d'une solution aqueuse de HF de préférence à 50 %.

Le temps de réaction, à température ambiante, est généralement compris entre environ 1 et 8 heures puis l'on décante la phase aqueuse et l'on récupère la phase organique qui est alors chauffée sous pression ambiante de facon à éliminer l'eau qu'elle contient par distillation de l'azéotrope "eau-solvant organique".

Le produit obtenu se présente le plus souvent sous forme visqueuse contenant une certaine proportion de solvant mais qui n'est pas nuisible pour les réactions de fluoration ultérieures.

Les fluorures d'ammonium de départ peuvent être préparés selon la méthode décrite par H. KOBLER et al. Liebigs Ann. Chem. (1978) 1937 selon le schéma réactionnel suivant:

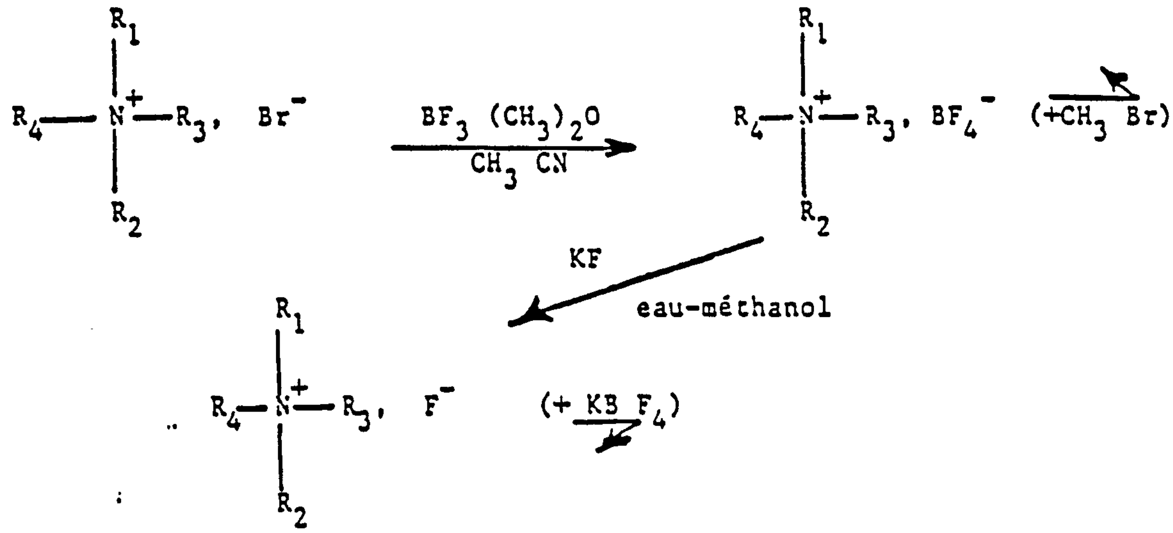


Le procédé de préparation des résines porteuses d'anions dihydrogénotrifluorures consiste à traiter une résine échangeuse d'ions telles qu'une résine Amberlyst A 26R ou Amberlite IRA 900R sous forme $Cl^-$ à l'aide de soude puis à l'aide d'une solution aqueuse de HF en vue de la mettre sous forme $F^-$.

La resine sous forme $F^-$ est alors traitée comme décrit ci-dessus et dans le même rapport molaire par une solution aqueuse saturée d'un mélange de fluorure d'hydrogène aqueux et d'un fluorure alcalin ou d'un hydrogénodifluorure alcalin ou d'ammonium.

Après agitation à la température ambiante pendant un temps d'environ 5 à 8 heures, la résine est filtrée puis lavée.

La présente invention a également pour objet un procédé de préparation de composés fluorovinyliques à partir de composés acétyléniques mono-activés ou bi-activés selon le schéma réactionnel suivant:

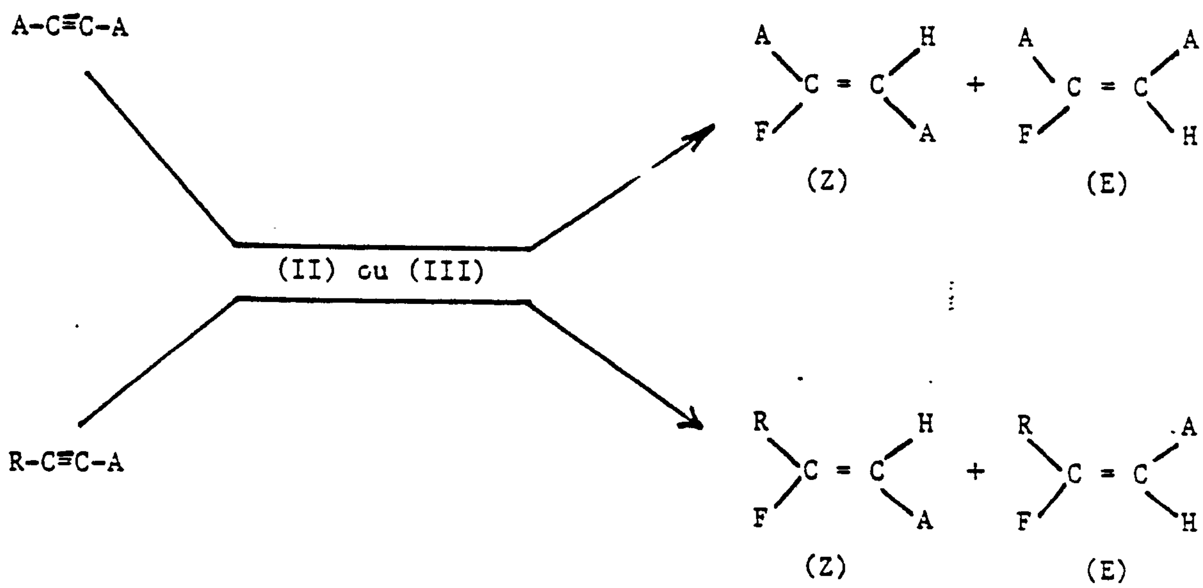


A = représentant une fonction activante et
R = alkyle, aryle ou aralkyle.

Ce procédé consiste à mettre en contact un composé acétylénique mono ou bi-activé en présence d'un agent de fluoration tel que défini ci-dessus à une température comprise entre la température ambiante et environ 120°C et pendant un temps compris entre environ 3 et 50 heures.

L'agent de fluoration est généralement utilisé en excés molaire d'environ 1,5 à 3 et de préférence d'environ 2 par rapport au composé acétylénique.

De préférence, la réaction est effectuée en présence d'un solvant organique peu polaire ou moyennement polaire en vue de solubiliser le composé acétylénique mais peut être réalisée éventuellement sans solvant

lorsque l'agent de fluoration est un dihydrogénotrifluorure d'ammonium de formule (II).

Comme solvant inerte vis à vis de l'agent de fluoration et du substrat acétylénique, on utilise préférentiellement un solvant chloré tel que le dichlorométhane, le dichloro-1,2 éthane, le tétrachlorure de carbone ou le chloroforme, ou un hydrocarbure aliphatique tel que le n-octane.

Après la fin de la réaction et refroidissement éventuel, le milieu réactionnel est traité à l'eau et extrait à l'éther ou tout simplement soumis à une filtration lorsque l'agent de fluoration est une résine porteuse d'anions dihydrogénotrifluorures.

Comme ceci a été indiqué précédemment, l'addition de HF s'arrête au stade éthylénique, le mélange éventuel des stéréo-isomères (Z) et (E) étant fonction de l'agent de fluoration et du substrat acétylénique de départ.

Par rapport aux dihydrogénotrifluorures d'ammonium (II), l'utilisation des résines porteuses d'anions dihydrogénotrifluorures semble favoriser plus nettement le syn-addition de HF.

Le tableau suivant permet de mettre en évidence cette propriété lorsque l'on utilise d'une part le dihydrogénotrifluorure de tétrabutylammonium $(C_4H_9)$ $N^+$, $H_2F_3^-$ et d'autre part, une résine Amberlyst A 26 porteuse d'anions $H_2F_3^-$.

| Composés fluorés | $(C_4H_9)_4N^+H_2F_3^-$ isomère Z | isomère E | Amberlyst A26 $H_2F_3^-$ isomère Z | isomère E |
|---|---|---|---|---|
| $CH_3O_2$-CF=CH-$CO_2CH_3$ | 100 | 0 | 81 | 19 |
| NC-CF=CH-CN | 63 | 37 | 68 | 32 |
| $C_7H_{15}$-CF=CH-CN | 70 | 30 | 33 | 67 |
| $C_6H_5$-CF=CH-CN | 80 | 20 | 72 | 28 |
| $C_4H_9$-CF=CH-$CO_2CH_3$ | 42 | 58 | 35 | 65 |
| $C_6H_5$-CF=CH-$CO_2CH_3$ | 95 | 5 | 88 | 12 |
| $C_6H_5$-CF=CH-CH=O | 91 | 9 | 83 | 17 |

On doit par ailleurs noter que l'on n'a observé aucune différence notable dans les rendements entre l'utilisation de ces deux types d'agent de fluoration.

Bien qu'il ait été fait plus, particulièrement référence aux propriétés d'addition de HF sur des acétyléniques, les agents de fluoration selon l'invention constituent également des composés particulièrement précieux pour accéder à des composés organo-fluorés par substitution nucléophile par l'anion $F^-$ sur carbone $sp_3$.

En particulier les résines porteuses d'anions $H_2F_3^-$ se sont avérées être de bons agents de fluoration d'α-bromo cétones et d'α-bromo esters, les taux de substitution sont en général très bons et obtenus dans des conditions douces.

Des réactions tout à fait similaires ont été également observées à l'aide de composés non activés.

On va maintenant donner à titre d'illustration et sans aucun caractère limitatif plusieurs exemples de préparation des agents de fluoration selon l'invention ainsi que des exemples de fluoration de composés acétyléniques.

Partie experimentale

A. Préparation du dihydrogènotrifluorure de tétrabutylammonium $(C_4H_9)_4N^+$, $H_2F_3^-$

1. Préparation du fluorure de tétrabutylammonium: $(C_4H_9)_4N^+F^-$

A 9,9 g (0,03 mole) de $(C_4H_9)_4N^+BF_4^-$, préparé selon H. KOBLER er al., Liebigs Ann. Chem. (1978), 1937, dissous dans 20 ml de méthanol, on ajoute 1,8 g (0,031 mole) de KF dissous dans 6 ml d'eau. Tous les ions $BF_4^-$ sont entraînés dans $KBF_4$ qui précipite que l'on filtre puis lave avec un peu de méthanol. La majeure partie du méthanol et de l'eau est ensuite éliminée par évaporation sous vide de la trompe à 40°C pendant environ une demi heure. On obtient un résidu liquide un peu visqueux, du produit attendu, accompagné d'un peu d'eau et de méthanol qui est utilisé tel quel pour la réaction suivante de transfert de phase.

Il n'est pas souhaitable d'éliminer totalement l'eau résiduelle car ceci peut entraîner une décomposition du produit.

2. Préparation du dihydrogènotrifluorure de tétrabutylammonium: $(C_4H_9)N^+$, $H_2F_3^-$

0,03 mole de $(C_4H_9)_4$ $N^+F^-$ tel que préparé ci-dessus est dissous dans 300 ml dichloro-1,2 éthane (DCE). A cette solution organique contenue dans un flacon en polyéthylène, on ajoute un solution aqueuse saturée soit d'un mélange HF-KF dans les proportions de 12 g HF aqueux à 50 % (0,3 mole) + 8,7 g de KF (0,15 mole) et 23 ml d'eau, soit d'un mélange HF-$KHF_2$ dans les proportions 6 g HF aqueux à 50 % (0,15 mole) + 11,7 g de $KHF_2$ (0,15 mole) et 35 ml d'eau.

Le mélange hétérogène de ces deux phases organique et aqueuse est laissé sous agitation à température ambiante pendant environ deux heures. Après décantation de la phase aqueuse, la phase organique récupérée est chauffée sous pression ambiante de façon à éliminer l'eau qu'elle contient par distillation de l'azéotrope

(eau - DCE), puis la majeure partie du DCE restant est finalement évaporée sous vide.

Le résidu visqueux obtenu de dihydrogènotrifluorure de tétrabutylammonium contient environ 15 % en poids de DCE dont la présence ne constitue aucune gêne pour les réactions de fluoration.

Par évaporation poussée du DCE, on obtient un échantillon de dihydrogènotrifluorure de tétrabutylammonium dont l'analvse élémentaire conduit au résultat suivant:

| | | | | |
|---|---|---|---|---|
| Cal. : | C % 63,74 | H % 12,7 | N % 4,65 | F % 18,91 |
| Tr. : | 61,56 | 12,13 | 4,42 | 18,64 |

Dosage acido-basique

Le dosage du résidu effectué par de la soude en présence de phénol-phtaléine permet de déduire le nombre n de mole de HF fixées par mole de $(C_4H_9)_4 N^+F^-$. Ce nombre est en moyenne compris entre 1,9 et 2,1 (ce calcul tenant compte de la quantité résiduelle de DCE difficile à éliminer totalement et évaluée par RMN$^1$H).

Spectre de RMN

En RMN $^1$H le spectre d'un échantillon du résidu obtenu dissous dans $CDCl_3$ fournit en plus des signaux caractéristiques du groupement $(C_4H_9)_4N^+$:

- Un singulet très fin à $\delta = 3,8$ ppm attribuable au DCE,
- Un singulet moyennement large ($\Delta\nu$ à mi-hauteur ~ $5H_z$)

dans la région des champs faibles qui, quel que soit l'échantillon, se caractérise par une intensité moyenne de 1,9 - 2,1 noyaux de H et par un déplacement chimique moyen $\delta = 12,9$ ppm (concentration ≃ 1,5 - 2 mol.l$^{-1}$). En RMN $^{19}$F on note un singulet à $\delta = -167$ ppm (mesuré par rapport à $CFCl_3$).

Spectre Infrarouge

Le spectre IR présente des bandes d'absorption attribuables respectivement aux vibrations de combinaison (2560 cm$^{-1}$ et 2320 cm$^{-1}$) d'élongation (1800 cm$^{-1}$) et de déformation (1155 cm$^{-1}$, 1115 cm$^{-1}$ et 1060-1050 cm$^{-1}$ caractéristiques de l'anion $H_2F_3^-$.

Préparation de composés fluorovinyliques à partir de composés acetyleniques mono et bi-actives à l'aide du dihydrogènotrifluorure de tétrabutylammomium $(C_4H_9)_4N^+H_2F_3^-$

1. Acétyléniques mono-activés

Mode opératoire général

Dans un ballon de 50 ml on ajoute 0,015 mole du composé acétylénique et 0,03 mole de dihydrogènotrifluorure de tétrabutylammonium. Le ballon est bouché, le mélange réactionnel est chauffé, sous agitation, au bain d'huile thermostaté à 110 ou 120° C selon les cas.

Le milieu réactionnel est ensuite traité à l'eau, extrait à l'éther puis la phase étherée est lavée à l'eau et séchée sur sulfate de sodiun anhydre. Après évaporation de l'éther, le résidu est purifié par distillation. On obtient ainsi l'adduit ou un mélange d'adduits stéréo-isomères (Z) et (E).

Selon le mode opératoire décrit ci-dessus on a ainsi traité les composés acétyléniques suivants:

$C_7H_{15}\text{-}C{\equiv}C\text{-}CN$ (1)
$C_6H_5\text{-}C{\equiv}C\text{-}CN$ (2)
$C_4H_9\text{-}C{\equiv}C\text{-}CO_2CH_3$ (3)
$C_6H_5\text{-}C{\equiv}C\text{-}CO_2CH$ (4)
$C_6H_5\text{-}C{\equiv}C\text{-}CO_2C_2H_5$ (5)
$C_6H_5\text{-}C{\equiv}C\text{-}COC_6H_5$ (6)
$C_6H_5\text{-}C{\equiv}C\text{-}CH{=}O$ (7)

Les résultats obtenus en composés fluorovinyliques sont rassemblés dans le tableau suivant:

| COMPOSES OBTENUS | Température (°C) | Durée de réaction (h) | Taux global de transformation (%) | Proportions relatives en stéréoisomères Z | E |
|---|---|---|---|---|---|
| (1') $C_7H_{15}$-CF=CH-CN | 110 | 7 | 95 | 70 | 30 |
| (2') $C_6H_5$-CF=CH-CN | 110 | 8 | 80 | 80 | 20 |
| (3') $C_4H_9$-CF=CH-$CO_2CH_3$ | 120 | 24 | 90 | 42 | 58 |
| (4') $C_6H_5$-CF=CH-$CO_2CH_3$ | 120 | 21 | 75 | 95 | 5 |
| (5') $C_6H_5$-CF=CH-$CO_2C_2H_5$ | 120 | 24 | 45 | 82 | 18 |
| (6') $C_6H_5$-CF=CH-CO-$C_6H_5$ | 110 | 50 | 53 | 100 | 0 |
| (7') $C_6H_5$-CF=CH-CHO | 100 | 4,5 | 75 | 91 | 9 |

Les caractéristiques des composés fluorovinyliques obtenus 1' à 7' sont donnés ci-après:

- (1') Fluoro - 3 décène - 2 nitrile (Z+E)
$Eb_{20}$ = 113 - 133°C - $R^{dt}$ 84 %.
Analyse: $C_{10}H_{16}FN$

| | C % | H % | F % | N % |
|---|---|---|---|---|
| Calc. : | 70,97 | 9,53 | 11,23 | 8,28 |
| Tr. : | 70,99 | 9,54 | 10,79 | 7,91 |

- (2') Fluoro - 3 phényl - 3 propène - nitrile (Z + E)
$Eb_{10}$ = 100 - 128°C - $R^{dt}$ 70 %.
Analyse: $C_9H_6FN$

| | C % | H % | F % | N % |
|---|---|---|---|---|
| Calc. : | 73,46 | 4,11 | 12,91 | 9,52 |
| Tr. : | 73,26 | 4,16 | 12,79 | 9,66 |

- (3') Mélange de fluoro - 3 heptène - 2 oate de méthyle $C_4H_9$-CF=CH-$CO_2CH_3$ (Z + E) et de fluoro - 3 heptène - 3 oate de méthyle $C_3H_7$-CH=CF-$CH_2$-$CO_2CH_3$ (Z + E) (prop. relatives 5/3): $Eb_{21}$ = 67 - 84°C.
Spectre de masse: $C_8H_{13}FO_2$ - (M+)

| | |
|---|---|
| Calc. : | 160,089950 |
| Tr. : | 160,0894 |

- (4') Fluoro - 3 phényl - 3 propénoate de méthyle (Z + E)
$Eb_{1,5}$ = 113 - 116°C - $R^{dt}$ 67 %.
Spectre de masse : $C_{10}H_9FO_2$ (M+)

| | |
|---|---|
| Calc. : | 180,058652 |
| Tr. : | 180,0588 |

- (5') Fluoro - 3 phényl - 3 propénoate d'éthyle (Z + E)
(caractérisé par IR et RMN mais non isolé)

- (6') Diphényl - 1,3 fluoro - 3 propénone (Z)
F = 61°C
Ce stéréo-isomère Z a été obtenu pur après chromatographie du mélange réactionnel sur gel de silice (éluant: Hexane - Benzène (70/30)).
Analyse: $C_{15}H_{11}FO$

| | C % | H % |
|---|---|---|
| Calc. : | 79,63 | 4,90 |
| Tr. : | 79,57 | 4,98 |

- (7') Fluoro - 3 phényl - 3 propénal (Z + E)
$Eb_{0,7}$ = 83°C - $R^{dt}$ 61 %
A partir de ce mélange distillé, le stéréo-isomère (Z) a été obtenu pur par lavage au pentane du mélange (Z + E): on récupère ainsi un solide bien cristallisé F = 32°C
Spectre de masse: $C_9H_7FO$: (M+)

| | |
|---|---|
| Calc. : | 150,048089 |
| Tr. : | 150,0482 |

Ce dernier composé est facilement oxydable en acide fluoro - 3 phényl - 3 propénoïque $C_6H_5$-CF=CH-COOH, qui a été caractérisé en spectrométrie de masse: $C_7H_7FO_2$: (M+ - H)

| | |
|---|---|
| Calc. : | 165,0334 |
| Tr. : | 165,0352 |

2. Acétyléniques bi-activés

Les composés acétyléniques suivants:
$CH_3O_2C$-C≡C-$CO_2CH_3$ (8)
$C_6H_5CO$-C≡C-$COC_6H_5$ (9)
CN-C≡C-CN (10)
traités dans les conditions telles que définies ci-après ont conduit aux résultats suivants en composés fluorovinyliques:

| COMPOSES OBTENUS | Température (°C) | Durée de réaction (heures ) | Taux global de transformation | Proportions relatives en stéréoisomères Z | E |
|---|---|---|---|---|---|
| (8') $CH_3O_2C$-CF=CH-$CO_2CH_3$ | 60 | 9 | 90 | 100 | 0 |
| (9') $C_6H_5CO$-CF=CH-CO-$C_6H_5$ | 60 | 24 | 57 | 100 | 0 |
| (10') NC-CF=CH-CN | 25 | 3 | 85 | 65 | 35 |

Préparation du composé de formule: $CH_3O_2C$-CF=CH-$CO_2CH_3$ (8')

A 0,03 mole de dihydrogènotrifluorure de tétrabutylammonium on ajoute, dans un ballon de 50 ml, 0,015 mole d'acétylène dicarboxylate de méthyle (8). Le ballon contenant ce mélange qui forme une solution homogène est bouché et chauffé sous agitation magnétique au bain d'huile thermostaté à 60°C. On observe un noircissement rapide du milieu réactionnel dés la formation du mélange. Des prélévements analysés en RMN $^1$H, permettent de suivre le cours de la réaction. Au bout de 9 heures de chauffage à 60°C, la fixation d'un équivalent de HF à la liaison acétylènique apparaît quasi-totale et la réaction n'évolue plus.

Après refroidissement, le milieu réactionnel est traité à l'eau et extrait à l'éther. La phase éthérée est lavée plusieurs fois à l'eau jusqu'à pH environ 5-6 et séchée sur sulfate de sodium anhydre. Après évaporation de l'éther, on récupère le fluorofumarate de méthyle (Z) (8') qui est purifié par distillation ($Eb_{10-11}$ = 92°C) ou par recristallisation dans un mélange éther-éther de pétrole (point de fusion 46°C). Rendement = 75 %.

Analyse élémentaire $C_6H_7FO_4$

| | | | |
|---|---|---|---|
| Calc. : | C % 44,45 | H % 4,35 | F % 11,71 |
| Tr. : | 44,52 | 4,36 | 10,76 |

2. Préparation du composé de formule: $C_6H_5$-CO-CF=CH-CO-$C_6H_5$ (9')

A 0,015 mole de dicétone acétylénique (9) dissoute dans 15 ml de dichloro-1,2 éthane, on ajoute 0,03 mole de dihydrogènotrifluorure de tétrabutylammonium. Ce mélange homogène est chauffé sous agitation dans un ballon de 50 ml, équipé d'un réfrigérant à reflux surmonté d'un tube à chlorure de calcium, durant 24 heures, au bain d'huile thermostaté à 60°C.

Après refroidissement le mélange est d'abord extrait à l'éther, puis lavé à l'eau et séché sur sulfate de sodium anhydre. Après évaporation des solvants, on récupère un mélange de dicétone initiale (9) et de l'adduit attendu.

La séparation de ces deux composés ne peut être obtenue que par chromatographie sur silice (éluant: hexane-dichlorométhane dans les proportions 80/20). On obtient ainsi la dicétone fluorée attendue sous la forme d'un solide jaune pile de point de fusion: 62°C.

Analyse élémentaire: $C_{16}H_{11}FO_2$

| | | | |
|---|---|---|---|
| Calc. : | C % 75,58 | H % 4,36 | F % 7,47 |
| Tr .: | 75,55 | 4,45 | 7,00 |

3. Préparation du composé de formule: NC-CF=CH-CN (10')

1 g (0,013 mole) d'acétylène dinitrile (10) est dissous dans 20 ml de dichloro-1,2 éthane. On ajoute à cette solution à température ambiante 0,026 mole de dihydrogènotrifluorure de tétrabutylammonium en une seule fois. Le mélange réactionnel noircit aussitôt et s'échauffe nettement (la température atteint environ 40° C). Ce mélange est ensuite laissé sous agitation à température ambiante durant 3 heures. Puis comme précedemment, on traite à l'éther puis à l'eau et la phase éthérée est lavée à l'eau et séchée sur sulfate de sodium anhydre. Après évaporation des solvants, on récupère un résidu dans lequel sont parfaitement caractérisés les adduits stéréoisomères (Z) et (E) par spectrométrie de masse et par RMN.

Spectre de masse: $C_4HFN_2$-(M+)

| | |
|---|---|
| Calc. : | 96,012374 |
| Tr. : | 96,0126 |

B. Preparation des résines Amberlyst à 26 et Amberlite IRA 900 porteuses d'anions $H_2F_3^-$

Les résines du commerce Amberlyst A 26 ou Amberlite IRA 900 se présentent sous forme $Cl^-$.

Dans une première étape elles sont transformées sous forme $F^-$ selon la méthode décrite par Colonna et al. J. Chem. Soc. Perkin I, (1979), 2248, c'est-à-dire par deux lavages successifs le premier à la soude diluée (≃1N) puis la seconde par une solution aqueuse de HF (≃1N).

50 g de résine Amberlyst A26 ou Amberlite IRA 900 sous forme F ce qui correspond à environ 0,1 mole d'anion, sont ajoutés dans un récipient en polyéthylène à une solution aqueuse constituée soit de 20g d'une solution aqueuse de HF à 50 % (0,5 mole) + 39 g (0,5 mole) de K $HF_2$ + 115 ml d'eau, soit de 40 g d'une solution aqueuse de HF à 50 % (1 mole) + 29 g (0,5 mole) de KF + 70 ml d'eau.

Après mélange à température ambiante pendant 8 heures, on filtre et on lave la résine par de l'acétone pour éliminer la majeure partie de l'eau qu'elle contient puis à l'éther. La résine séche obtenue contient encore un peu d'eau et est ajoutée à 400 ml de dichloro-1,2 éthane (DCE). L'eau restante est alors éliminée par distillation de l'azéotrope eau + DCE.

Après filtration et séchage à l'air, la résine est finalement récupérée et utilisée telle quelle dans les réactions ultérieures.

Caractéristiques des résines obtenues

La capacité d'échange ionique de ces résines en matière sèche vaut en moyenne selon la littérature 3,8 meq $g^{-1}$ sous forme $Cl^{-1}$.

Afin d'évaluer le nombre n de moles de HF transférées on a tout d'abord déterminé d'après C. Cainelli et al. Synthesis (1976) 472, la capacité d'échange de ces résines (matière sèche) sous forme $OH^-$ par un dosage acido-basique à l'acide sulfurique.

Ensuite à l'issue du traitement de la forme F pour accéder à la forme ($F^-$, n HF) on a dosé l'acidité de cette dernière forme par la soude en présence du phénol phtaléïne.

Les résultats obtenus ont été les suivants:

| Résine | Forme $OH^-$ nombre de meq $g^{-1}$ | Forme [$F^-$, nHF] nombre de meq $g^{-1}$ | n |
|---|---|---|---|
| Amberlyst A 26 | 4,0 - 4,1 | 6,7 - 6,9 | 1,95 - 2,04 |
| Amberlite IRA 900 | 4,0 - 4,2 | 6,9 - 7,2 | 1,95 - 2,05 |

On détermine la valeur du nombre n en fonction des considérations suivantes:

a) à partir des résultats issus des dosages de résines sous forme $OH^-$ on peut déduire pour chaque résine une masse molaire associée à un équivalent d'ion $OH^-$. On trouve ainsi M = 233 - 236 g pour la résine Amberlyst A 26 et M = 221 - 233 g pour la résine Amberlite IRA 900.

b) on admet que la forme $OH^-$ est ensuite entièrenent neutralisée par HF, de sorte que le dosage des formés [$F^-$, n HF] puisse être exploité en utilisant les valeurs des masses molaires fictives trouvées ci-dessus. On arrive ainsi à une estimation convenable et très reproductible, du nombre n cherché qui vaut en moyenne pour ces résines de 1,9 à 2,1.

Préparation de composés fluorovinyliques à partir de composés acetyleniques mono et bi-actives à l'aide de résines Amberlyst A 26 et Amberlite IRA 900 d'anions $H_2F_3^-$.

1. Acétyléniques mono-activés

Mode opératoire général

Dans un ballon de 50 ml contenant 0,015 mole de composé acétylénique dissous dans 20 ml de n -octane, on ajoute 9 g de résine Amberlyst A 26 ou Amberlite IRA 900 porteuse d'anions $H_2F_3^-$ telles qu'obtenues ci-dessus. Le ballon est équipé d'un réfrigérant à reflux surmonté d'un tube à chlorure de calcium et le milieu réactionnel est chauffé sous agitation au bain d'huile thermostaté à 110 - 120°.

Après refroidissement du milieu réactionnel la résine est filtrée et lavée à l'éther. Après évaporation des solvants, le résidu est analysé en RMN $^1H$ pour déterminer le rendement global de l'addition de HF ainsi que les porportions relatives des stéréoisoméres. Ensuite les composés fluorovinyliques obtenus sont purifiés par distillation du résidu.

Les composés fluorovinyliques sont parfaitement identifiés par leurs constantes physiques et leurs paramètres spectroscopiques par comparaison avec les valeurs trouvées précédemment à l'issue des réactions avec le dihydrogénotrifluorure de tétributylammonium.

Selon le mode opératoire décrit ci-dessus on a ainsi traité les composés acétyléniques suivants:

$C_7H_{15}$-C≡C-CN (1)
$C_6H_5$-C≡C-CN (2)
$C_4H_9$-C≡C-$CO_2CH_3$ (3)
$C_6H_5$-C≡C-$CO_2CH_3$ (4)
$C_6H_5$-C≡C-CH=O (7)

Les résultats obtenus en composés fluorovinyliques sont rassemblés dans le tableau suivant:

| COMPOSES OBTENUS | Température (°C) | Durée de réaction (heures) | Taux global de transformation (%) | Proportions relatives en stéréoisomères Z | E |
|---|---|---|---|---|---|
| (1') $C_7H_{15}$-CF=CH-CN | 110 | 30 | 95 | 33 | 67 |
| (2') $C_6H_5$-CF=CH-CN | 110 | 20 | 88 | 72 | 28 |
| (3') $C_4H_9$-CF=CH-$CO_2CH_3$ | 110 | 16 | 78 | 35 | 65 |
| (4') $C_6H_5$-CF=CH-$CO_2CH_3$ | 120 | 24 | 85 | 88 | 12 |
| (7') $C_6H_5$-CF=CH-CH=O | 110 | 4 | 62 | 83 | 17 |

2. Acétyléniques bi-activés

Les composés acétyléniques suivants:

$CH_3O_2C$-C≡C-$CO_2CH_3$ (8)
CN-C=C-CH (10)

traités dans les conditions telles que décrites ci-après ont conduit aux résultats suivants en composés fluorovinyliques:

| COMPOSES OBTENUS | Température (°C) | Durée de réaction (heures) | Taux global de transformation | Proportions relatives en stéréoisomères Z | E |
|---|---|---|---|---|---|
| (8') $CH_3O_2C$-CF=CH-$CO_2CH_3$ | 60 | 30 | 80 | 81 | 19 |
| (10') NC-CF=CH-CN | 25 | 7 | 87 | 68 | 32 |

Préparation du composé de formule: $CH_3O_2C$-CF=CH-$CO_2CH_3$

Dans un ballon de 50 ml, on ajoute successivement 0,015 mole (2,1 g) d'acétylène dicarboxylate de méthyle, 8,8 g de résine Amberlyst A 26 porteuse d'anions $H_2F_3^-$ telle qu'obtenue ci-dessus (ce qui équivaut à environ 0,03 mole de $H_2F_3^-$) et 10 ml de tétrachlorure de carbone. Le ballon est équipé d'un réfrigérant à reflux surmonté d'un tube à $Cl_2$ Ca et le milieu réactionnel est chauffé au bain d'huile thermostaté à 60°C. La réaction est suivie par l'analyse de prélévements en RMN $^1H$. Lorsque la réaction est terminée, on filtre la résine on la lave à l'éther et on évapore les solvants pour récupérer le produit attendu (1,7 soit un rendement de 70 %).

SPECTRE RMN du stéréoisomère (E)

En RMN$^1$ : = CH: $\delta$ = 6,09 (d) - $^3$JHF = 16, OHz

19F : = CF: $\delta$ = -109,4 (d) - $^3$JHF = 15,7 Hz

Préparation du composé de formule: NC-CF=CH-CN

1 g (0 013 mole d'acétylène dinitrile est dissous dans 20 ml de tétrachlorure de carbone. A cette solution dans un ballon de 50 ml on ajoute 7,7 g de résine Amberlyst A 26 porteuse d'anions $H_2F_3^-$. Le ballon est bouché puis le mélange est laissé sous agitation à température ambiante durant 7 heures. La résine est lavée puis filtrée à l'éther et les solvants sont évaporés.

On récupère ainsi un résidu contenant un mélange des stéréoisomères (E) et (Z) parfaitement caractérisés par leurs spectres RMN.

**Revendications**

1. Agent de fluoration, notamment de composés acétyléniques, caractérisé par le fait qu'il répond à la formule générale suivante:

$$Q^+ \ [F^-, n \ HF] \qquad (I)$$

dans laquelle:

$Q^+$ représente soit: (i) un cation de formule:

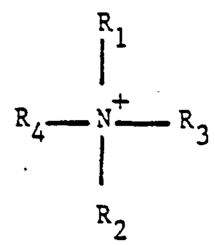

dans laquelle:

$R_1$, $R_2$, $R_3$ et $R_4$, identiques ou différents, représentent un radical alkyle ayant de 1 à 20 atomes de carbone, un radical aryle ou un radical aralkyle, le nombre d'atomes de carbone dans $R_1+R_2+R_3+R_4$ étant au moins égal à 13 et de préférence compris entre 15 et 22,

soit: (ii) une matrice polymérique porteuse de fonctions de formule:

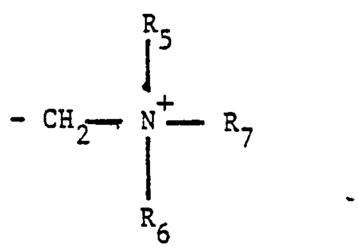

dans laquelle:

$R_5$ $R_6$ et $R_7$ identiques ou différents, représentent un radical alkyle inférieur ayant de 1 à 6 atomes de carbone,

et n est une valeur moyenne comprise entre 1,5 et 2,5 de préférence entre 1,8 et 2,1.

2. Agent de fluoration selon la revendication 1, caractérisé par le fait qu'il répond à la formule suivante:

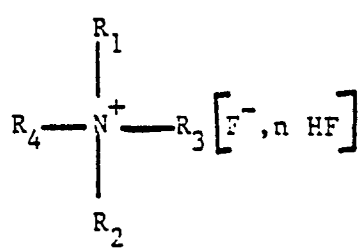

(II)

dans laquelle:

$R_1$, $R_2$, $R_3$ et $R_4$ identiqués ou différents, représentent un ridical alkyle ayant de 1 à 20 atomes de carbone, un radical phényle ou un radical benzyle, le nombre total d'atomes de cirbone des radicaux $R_1+R_2+R_3+R_4$ étant au moins égal à 13 et de préférence compris entre 15 et 22,

et n est compris entre 1,5 et 2,5 et de préférence entre 1,8 et 2,1.

3. Agent de fluoration selon l'une quelconque des revendications 1 et 2, caractérisé par le fait qu'il est le dihydrogènotrifluorure de tétrabutylammonium,

le dihydrogènotrifluorure de tétrapentylammonium,
le dihydrogènotrifluorure de tétrahéxylammonium,
le dihydrogènotrifluorure d'hexadécyltriméthylammonium,
le dihydrogènotrifluorure de benzyltriéthylammonium.

4. Agent de fluoration selon l'une quelconque des revendications précédentes, caractérisé par le fait que l'agent de fluoration est le dihydrogénotrifluorure de tétrabutylammonium.

5. Agent de fluoration selon la revendication 1, caractérisé par le fait qu'il répond à la formule suivante:

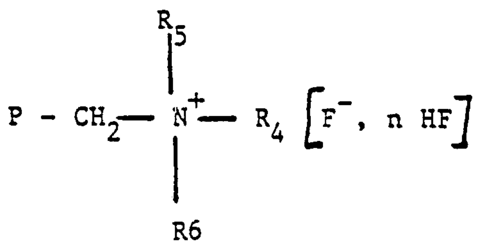

(III)

dans laquelle:

$R_4$, $R_5$ et $R_6$, identiques ou différents, réprésentent un radical alkyle inférieur ayant de 1 à 6 atomes de carbone et P représente une matrice polymérique, et n est compris entre 1,5 et 2,5 de préférence entre 1,8 et 2,1.

6. Agent de fluoration selon la revendication 5, caractérisé par le fait que la matrice polymérique est du type polystyrène-divinylbenzène.

7. Procédé de préparation de l'agent de fluoration selon l'une quelconque des revendications 1 à 6, caractérisé par le fait qu'il consiste à mettre en contact un fluorure de formule: $Q^+F^-$ dans laquelle $Q^+$ est tel que défini à la revendication 1, en présence d'une solution aqueuse saturée d'un mélange de fluorure d'hydrogène aqueux et d'un fluorure alcalin ou d'un hydrogènodifluorure alcalin ou d'ammonium, le rapport molaire des constituants de la solution aqueuse par rapport au fluorure $Q^+F^-$ étant compris entre environ 4 et 6.

8. Procédé selon la revendication 7, caractérisé par le fait qu'il est réalisé selon la technique de transfert de phase et consiste à faire réagir une phase organique constituée d'un solvant moyennement polaire contenant un fluorure d'ammonium de formule:

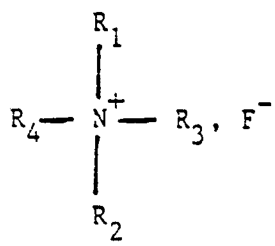

dans laquelle:

$R_1$, $R_2$, $R_3$ et $R_4$ ont les mêmes significations que celles données à la revendication 1,

en présence d'une phase aqueuse saturée d'un mélange de fluorure d'hydrogène aqueux et d'un fluorure alcalin ou d'un hydrogènodifluorure alcalin ou d'ammonium.

9. Procédé selon la revendication 8, caractérisé par le fait que la concentration du fluorure d'ammonium dans la phase organique est comprise entre 0,08 et 0,12 mole/l de solvant.

10. Procédé selon l'une quelconque des revendications 7 à 9, caractérisé par le fait que la réaction est effectuée à température ambiante pendant un temps compris entre environ 1 et 8 heures.

11. Procédé de préparation de composés fluorovinyliques à partir de composés acétyléniques, caractérisé par le fait qu'il consiste à mettre en contact un composé acétylénique mono ou bi-activé en présence d'un agent de fluoration tel que revendiqué selon l'une quelconque des revendications 1 à 6.

12. Procédé selon la revendication 11, caractérisé par le fait qu'il est réalisé à une température comprise entre la température ambiante et environ 120° C pendant un temps compris entre environ 3 et 50 heures.

13. Procédé selon l'une quelconque des revendications 11 et 12, caractérisé par le fait que la réaction est effectuée en présence d'un solvant organique peu polaire ou moyennement polaire pris dans le groupe constitué par le dichlorométhane, le dichloro-1,2 éthane, le tétrachlorure de carbone, le chloroforme et le n-octane.

14. Procédé selon l'une quelconque des revendication 11 à 13, caractérisé par le fait que l'agent de fluoration est utilisé en excés molaire d'environ 1,5 à 3 et de préférence d'environ 2 par rapport au composé acétylénique.

15. Utilisation de l'agent de fluoration selon l'une quelconque des revendications 1 à 6 pour l'obtention de composés organofluorés par substitution nucléophile.

**Patentansprüche**

1. Fluorierungsmittel, insbesondere für Acetylenverbindungen der folgenden allgemeinen Formel:

$Q^{+} [F^{-}, n\ HF]$ (I)

worin
$Q^{+}$ weder (i) ein Kation der Formel:

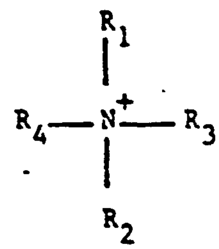

bedeutet, worin
$R_1$, $R_2$, $R_3$ und $R_4$ gleich oder verschieden sind und für einen Alkylrest mit 1 bis 20 Kohlenstoffatomen, einen Aryl- oder einen Aralkylrest stehen, wobei die zahl der Kohlenstoffatome von $R_1 + R_2 + R_3 + R_4$ mindestens 13, vorzugsweise zwischen 15 und 22, beträgt,
oder (ii) eine Polymermatrix mit Funktionen der Formel:

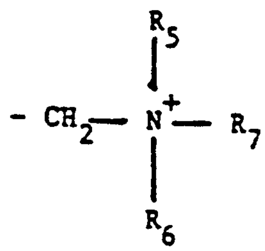

bedeutet, worin
$R_5$, $R_6$ und $R_7$ gleich oder verschieden sind und für einen Niedrigalkylrest mit 1 bis 6 Kohlenstoffatomen stehen, und
n einen Mittelwert von 1,5 - 2,5, vorzugsweise von 1,8 - 2,1 bedeutet.

2. Fluorierungsmittel nach Anspruch 1 der folgenden Formel:

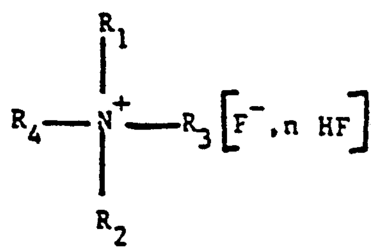
(II)

worin
$R_1$, $R_2$, $R_3$ und $R_4$ gleich oder verschieden sind und für einen Alkylrest mit 1 bis 20 Kohlenstoffatomen, einen Phenyl- oder einen Benzylrest stehen, wobei die Gesamtzahl der Kohlenstoffatome von $R_1 + R_2 + R_3 + R_4$ mindestens 13, vorzugsweise zwischen 15 und 22, beträgt, und
n zwischen 1,5 und 2,5, vorzugsweise zwischen 1,8 und 2,1 beträgt.

3. Fluorierungsmittel nach einem der Ansprüche 1 und 2, wobei es sich dabei um:
- Tetrabutylammoniumdihydrogentrifluorid,
- Tetrapentylammoniumdihydrogentrifluorid,
- Tetrahexylammoniumdihydrogentrifluorid,
- Hexadecyltrimethylammoniumdihydrogentrifluorid,
- Benzyltriethylammoniumdihydrogentrifluorid

handelt.

4. Fluorierungsmittel nach einem der vorhergehenden Ansprüche, wobei es sich um Tetrabutylammoniumdihydrogentrifluorid handelt.

5. Fluorierungsmittel nach Anspruch 1 der folgenden Formel:

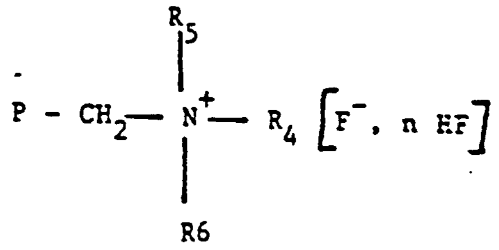 (III)

worin

$R_4$, $R_5$ und $R_6$ gleich oder verschieden sind und für einen Niedrigalkylrest mit 1 bis 6 Kohlenstoffatomen stehen,

p eine polymere Matrix bedeutet, und

n zwischen 1,5 und 2,5, vorzugsweise zwischen 1,8 und 2,1 beträgt.

6. Fluorierungsmittel nach Anspruch 5, dadurch gekennzeichnet, daß die polymere Matrix vom Polystyran-Divinylbenzol-Typ ist.

7. Verfahren zur Herstellung des Fluorierungsmittels nach einem der Ansprüche 1 bis 6, dadurch gekennzeichnet, daß ein Fluorid der Formel: $Q+ F^-$, worin Q+ die in Anspruch 1 definierte Bedeutung besitzt, in Anwesenheit einer gesättigten wäßrigen Lösung einer Mischung aus wäßrigem Fluorwasserstoff und einem Alkalifluorid oder einem Alkali- oder Ammoniumhydrogendifluorid in Kontakt gebracht wird, wobei das Molverhältnis der Komponenten der wäßrigen Lösung, bezogen auf das Fluorid $Q+ F^-$, zwischen etwa 4 und 6 beträgt.

8. Verfahren nach Anspruch 7, dadurch gekennzeichnet, daß es nach der Phasentransfer-Technik durchgeführt wird, wobei eine organische Phase, welche aus einem Lösungsmittel mittlerer Polarität besteht und ein Ammoniumfluorid der Formel:

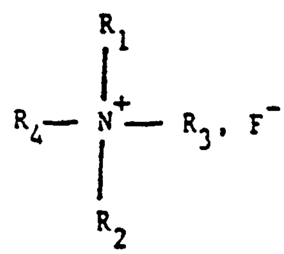

enthält, worin $R_1$, $R_2$, $R_3$ und $R_4$ die in Anspruch 1 gegebenen Bedeutungen besitzen, in Anwesenheit einer gesättigten wäßrigen Phase aus einer Mischung aus wäßrigem Fluorwasserstoff und einem Alkalifluorid oder einem Alkali- oder Ammoniumhydrogendifluorid zur Reaktion gebracht wird.

9. Verfahren nach Anspruch 8, dadurch gekennzeichnet, daß daß die Konzentration des Ammoniumfluorids in der organischen Phase zwischen 0,08 und 0,12 Mol/l Lösungsmittel beträgt.

10. Verfahren nach einem der Ansprüche 7 bis 9, dadurch gekennzeichnet, daß die Reaktion bei Umgebungstemperatur während eines zeitraumes von ca. 1 und 8 Stunden durchgeführt wird.

11. Verfahren zur Herstellung von Fluorvinylverbindungen aus Acetylenverbindungen, dadurch gekennzeichnet, daß eine mono- oder bis-aktivierte Acetylenverbindung in Anwesenheit eines in einem der Ansprüche 1 bis 6 beanspruchten Fluorierungsmittel in Kontakt gebracht wird.

12. Verfahren nach Anspruch 11, dadurch gekennzeichnet, daß es bei einer Temperatur zwischen Umgebungstemperatur und ca. 120°C während eines zeitraumes von ca. 3 bis 50 Stunden durchgeführt wird.

13. Verfahren nach einem der Ansprüche 11 und 12, dadurch gekennzeichnet, daß die Reaktion in Anwesenheit eines organischen Lösungsmittels mit geringer oder mittlerer Polarität, ausgewählt unter Dichlormethan, 1,2-Dichlorethan, Tetrachlorkohlenstoff, Chloroform und n-Octan, durchgeführt wird.

14. Verfahren nach einem der Ansprüche 11 bis 13, dadurch gekennzeichnet, daß das Fluorierungsmittel in einem Molüberschuß von ca. 1,5 bis 3, vorzugsweise ca. 2, bezogen auf die Acetylenverbindung, verwendet wird.

15. Verwendung des Fluorierungsmittels nach einem der Ansprüche 1 bis 6 zur Gewinnung fluorierter organischer Verbindungen durch nucleophile Substitution.

**Claims**

1. Fluorinating agent, particularly for acetylenic compounds, characterized in that it corresponds to the following general formula:

$$Q+ [F^- n HF] \quad (I)$$

in which:

Q+ denotes either: (i) a cation of formula:

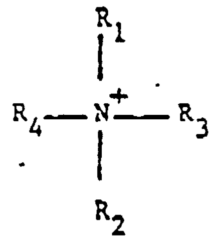

in which:

$R_1$, $R_2$, $R_3$ and $R_4$, which are identical or different, denote an alkyl radical containing from i to 20 carbon atoms, an aryl radical or an aralkyl radical, the number of carbon atoms in $R_1 + R_2 + R_3 + R_4$ being at least 13 and preferably between 15 and 22,

or: (ii) a polymeric matrix carrying functions of formula:

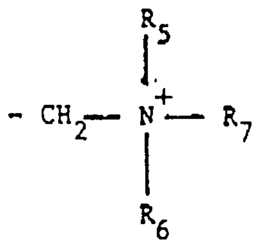

$R_5$, $R_6$ and $R_7$, which are identical or different, denote a lower alkyl radical containing 1 to 6 carbon atoms, and

n is a mean value of between 1.5 and 2.5, preferably between 1.8 and 2.1.

2. Fluorinating agent according to Claim 1, characterized in that it corresponds to the following formula:

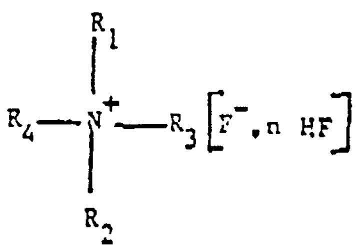

(II)

in which:

$R_1$, $R_2$, $R_3$ and $R_4$, which are identical or different, denote an alkyl radical containing from 1 to 20 carbon atoms, a phenyl radical or a benzyl radical, the total number of carbon atoms of the radicals $R_1 + R_2 + R_3 + R_4$ being at least 13 and preferably between 15 and 22, and

n is between 1.5 and 2.5 and preferably between 1.8 and 2.1.

3. Fluorinating agent according to either of Claims 1 and 2, characterized in that it is:
tetrabutylammonium dihydrotrifluoride,
tetrapentylammonium dihydrotrifluoride,
tetrahexylammonium dihydrotrifluoride,
hexadecyltrimethylammonium dihyrodtrifluoride,
benzyltriethylammonium dihydrotrifluoride.

4. Fluorinating agent according to any one of the preceding claims, characterized in that the fluorinating agent is tetrabutylammonium dihydrotrifluoride.

5. Fluorinating agent according to Claim 1, characterized in that it corresponds to the the following formula:

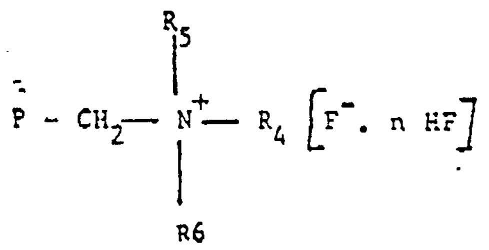 (III)

in which $R_4$, $R_5$ and $R_6$, which are identical or different, denote a lower alkyl radical containing from 1 to 6 carbon atoms and P denotes a polymeric matrix, and n is between 1.5 and 2.5, preferably between 1.8 and 2.1

6. Fluorinating agent according to Claim 5, characterized in that the polymeric matrix is of the polystyrene-divinylbenzene type.

7. Process for the preparation of the fluorinating agent according to any one of claims 1 to 6, characterized in that it consists in bringing into contact a fluoride of formula: $Q^+ F^-$ in which $Q^+$ is such as defined in Claim 1, in the presence of a saturated aqueous solution of a mixture of aqueous hydrogen fluoride and of an alkali metal fluoride or of an alkali metal or ammonium hydrodifluoride, the molar ratio of the constituents of the aqueous solution relative to the fluoride $Q^+ F^-$ being between approximately 4 and 6.

8. Process according to Claim 7, characterized in that it is carried out according to the phase transfer technique and consists in reacting an organic phase, consisting of a moderately polar solvent containing an ammonium fluoride of formula:

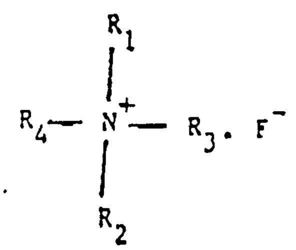

in which: $R_1$, $R_2$, $R_3$ and $R_4$ have the same meanings as those given in Claim 1,

in the presence of an aqueous phase saturated with a mixture of aqueous hydrogen fluoride and of an alkali metal fluoride or of an alkali metal or ammonium hydrodifluoride.

9. Process according to Claim 8, characterized in that the concentration of ammonium fluoride in the organic phase is between 0.08 and 0.12 mole/l of solvent.

10. Process according to any one of Claims 7 to 9, characterized in that the reaction is carried out at ambient temperature for a time of between 1 and 8 hours.

11. Process for the preparation of fluorovinyl compounds from acetylenic compounds, characterized in that it consists in bringing into contact a mono- or diactivated acetylenic compound in the presence of a fluorinating agent such as claimed according to any one of Claims 1 to 6.

12. Process according to Claim 11, chararterized in that it is carried out at a temperature of between the ambient temperature and approximately 120° C for a time of between approximately 3 and 50 hours.

13. Process according to either of Claims 11 and 12, characterized in that the reaction is carried out in the presence of a relatively non-polar or moderately polar organic solvent taken from the group consisting of dichloromethane, 1,2-dichloroethane, carbon tetrachloride, chloroform and n-octane.

14. Process according to any one of Claims 11 to 13, characterized in that the fluorinating agent is employed in a molar excess of approximately 1.5 to 3 and preferably of approximately 2 relative to the acetylenic compound.

15. Use of the fluorinating agent according to any one of Claims 1 to 6 to obtain organofluorinated compounds by nucleophilic substitution.